# EUROPÄISCHE PATENTSCHRIFT

(11) **EP 0 795 544 B1**
(45) Veröffentlichungstag und Bekanntmachung des Hinweises auf die Patenterteilung: **31.10.2001**
(21) Anmeldenummer: 97103445.9
(22) Anmeldetag: 03.03.1997
(51) Int. Cl.: C07C 263/04

(54) **Verfahren zur thermischen Spaltung von Carbamidsäureestern**
Process for the thermal decomposition of carbamic esters
Procédé de décomposition thermique d'esters carbamiques

(30) Priorität: 15.03.1996 DE 19610261; 10.05.1996 DE 19618828
(43) Veröffentlichungstag der Anmeldung: 17.09.1997
(73) Patentinhaber: BAYER AG, 51368 Leverkusen (DE)
(72) Erfinder: Dahmer, Jürgen, Dr., 47800 Krefeld (DE); Schleenstein, Dieter, Dr., 51519 Odenthal (DE); Steude, Heinrich, 51375 Leverkusen (DE); Wilmes, Oswald, Dr., 51061 Köln (DE); Rasp, Christian, Dr., 51467 Bergisch Gladbach (DE); Ronge, Georg, Dr., 40227 Düsseldorf (DE); Nachtkamp, Klaus, Dr., 40593 Düsseldorf (DE); Litz, Wilfried, Dr., 51107 Köln (DE); Kabelac, Stephan, Dr., 31515 Wunstdorf (DE)

(56) Entgegenhaltungen:
- EP-A- 0 092 738
- EP-A- 0 542 106
- EP-A- 0 568 782

## Beschreibung

Die Erfindung betrifft ein Verfahren zur thermischen Spaltung von Carbamidsäureestern in die korrespondierenden Isocyanate und die Hydroxylkomponenten in der Flüssigphase, wobei die Reaktion im Abtriebsteil einer Kolonne als Reaktivrektifikation abläuft und ein inertes schwersiedendes Lösemittel als Puffer die Reaktion aus dem Verdampferbereich der Kolonne fernhält.

Es ist bekannt die Spaltung von Carbamidsäureestern zu Isocyanaten in Gas- und Flüssigphasen sowie in der Wirbelschicht durchzuführen, z.B. in EP-A 28 724, EP-A 100 047, EP-A 126 299, EP-A 126 300, EP-A 143 120, EP-A 261 604, EP-A 449 110, US-A 3 734 941 und US-A 3 870 739.

Die Spaltung in der Gasphase ist ein Hochtemperatur-Verfahren, was in der Regel bei Temperaturen >300°C im Vakuum <25 mbar durchgeführt wird. Der hiermit verbundene verfahrenstechnische Aufwand, die thermische Belastung der Edukte und Produkte, die notwendige Vorab-Verdampfung des Carbamidsäureesters sowie die zum Teil noch ungeklärten katalytischen Effekte von Metalloberflächen bewirken, daß die Gasphasenspaltung gegenüber der Spaltung in der Flüssigphase nachteilig ist. Insbesondere besteht eine Verstopfungsgefahr im Verdampferbereich durch Verkrustung, da das Problem der Ausschleusung höhermolekularer Folgeprodukte nicht gelöst ist.

Die Spaltung in der Wirbelschicht wird z.B. in der EP-A 78 005 beschrieben. Dieses Verfahren ist mit hohem Energieeinsatz behaftet und läßt Schwierigkeiten bei der technischen Realisierung erwarten, so daß ein Einsatz aufgrund des vorläufigen Entwicklungsstandes im technischen Maßstab nicht absehbar ist.

Die Spaltung in der Flüssigphase ermöglicht im Vergleich zur Gasphasenspaltung niedrigere Reaktionstemperaturen von <300°C, erfordert aber das schnelle Abtrennen der Reaktionsprodukte. Zum einen wird dadurch die Rückreaktion von Isocyanat und Hydroxylkomponente zum Carbamidsäureester vermieden, zum anderen wird die Bildung von harzartigen Nebenprodukten, die zu Ablagerungen in den Apparaten führen können, vermindert bzw. vermieden. Eine Reduzierung der Reaktionstemperatur gelingt durch Katalysatorzugabe. Durch Verdünnen mit einem inerten Lösemittel kann die Entstehung höhermolekularer Folgeprodukte vermindert werden, gleichzeitig dient das Lösemittel zur Ausschleusung dieser Nebenkomponenten. Bei den Katalysatoren wirken sich nachteilig zum einen die verstärkten Nebenreaktionen, zum anderen die potentielle Verunreinigung des Produkts aus. Die nachfolgend aufgeführten Verfahren können nach Art des Reaktors in Rührreaktor bzw. Dünnschichter, in Reaktor mit aufgesetzter Kolonne und in Reaktionskolonnen unterschieden werden. Ein weiteres Unterscheidungsmerkmal ist die Gegenwart oder der Verzicht auf ein Lösemittel bei der Spaltreaktion.

Ein lösemittelfreies Spaltverfahren mit Katalysator ist aus der EP-A 524 554 ebenfalls für einen Reaktor mit aufgesetzter Kolonne bekannt. Der hier zugrundeliegende speziell gestaltete Reaktor ermöglicht die Spaltung in einem zweiphasigen Gemisch mit >50 % volumetrischem Gasgehalt. Hier wird durch Rektifikation eine Trennung ohne nennenswerte Rückreaktion erreicht wie beim Einsatz von Dephlegmatoren. Bei diesem Spaltreaktor ist zum einen ein großes Bauvolumen, zum anderen die Gefahr der Belegung von trockenen Heizflächenbereichen, insbesondere bei Abweichungen vom Nennlastzustand, zu erwarten. Die Reaktion findet hier unmittelbar im Bereich der Heizflächen statt, wo starke Temperaturgradienten und schlecht definierte Verweilzeiten vorliegen.

Ebenfalls eine Spaltkolonne ist Inhalt der Anmeldung EP-A 542 106. Hier wird die Spaltung ohne Katalysator in Gegenwart eines schwersiedenden Lösemittels in einer als Spaltreaktor dienenden Destillationskolonne beschrieben. Die Spaltprodukte werden durch Dephlegmatoren voneinander getrennt, die Zuspeisung des Carbamidsäureesters erfolgt im unteren Zeil der Kolonne. Es gelingt jedoch nur eine unbefriedigende Trennung zwischen dem Alkohol und dem Roh-Isocyanat.

Darüber hinaus findet in dem gemäß EP-A 542 106 beschriebenen Verfahren die Spaltung zum großen Teil im Sumpf der Kolonne statt, ein Umstand, der sich dadurch äußert, daß im Sumpf der Kolonne noch Carbamidsäureester sowie Nebenprodukte zu finden sind, obwohl der Sumpf durch sein gegenüber der Destillationskolonne erhöhtes Temperaturniveau sowie durch seine hohe Verweilzeit günstige Bedingungen für das Abreagieren bietet. Somit gelingt es mit diesem Verfahren nicht, die Spaltung vollständig durchzuführen mit der Folge, daß reagierende Nebenprodukte im Sumpf zu Anbackungen führen können, daß durch die Nebenprodukte Ausbeuteverluste entstehen und daß die nachfolgende Aufarbeitung erschwert ist. Es ist bekannt, daß aus Folgeprodukten entstehende Anbackungen die Standzeiten von Anlagen stark reduzieren können.

Die bekannten Verfahren zur Spaltung von Carbamidsäureestern in der Flüssigphase sind gegenüber den Gasphasenverfahren prinzipiell von Vorteil, da hier die hohe thermische Belastung der Reaktionsprodukte reduziert wird. Bei der Spaltung in der Flüssigphase ohne Lösemittel bereitet der schonende Energieeintrag für die stark endotherme Spaltung in der flüssigen Phase und das schnelle Entfernen der thermisch empfindlichen Spaltprodukte aus der "heißen" Zone noch Schwierigkeiten. Ein Nachteil der obengenannten Verfahren ist die überwiegende Durchführung der Spaltreaktion im beheizten Reaktor bzw. im Verdampferbereich der Spaltkolonne. An den beheizten Flächen finden bevorzugt die Ablagerungen statt, insbesondere wenn kein Lösemittel eingesetzt wird. Ein weiterer Nachteil bei den Verfahren ohne Lösemittel sind die hohen Ausschleusungsraten, die zur Reduzierung der Verweilzeit im Sumpf und zum Austragen der Nebenprodukte notwendig sind.

Aufgabe der vorliegenden Erfindung war es, ein Verfahren zur kontinuierlichen Spaltung von Carbamidsäureestern mit inertem Lösemittel und gleichzeitiger, kontinuierlicher, schneller Trennung der Spaltprodukte vom Edukt und Trennung der Spaltprodukte (Reaktivrektifikation) untereinander zu finden. Das Verfahren muß im technischen Maßstab wirtschaftlich mit hoher Raum-Zeit-Ausbeute über vernünftige Zeiträume betrieben werden können, ohne daß deren Funktion z.B. durch die Bildung von polymeren Ablagerungen deutlich behindert wird.

Diese Aufgabe wurde erfindungsgemäß dadurch gelöst, daß die Spaltreaktion im Abtriebsteil einer Kolonne in Form einer Reaktivrektifikation durchgeführt wird, wobei durch das Gegenstromprinzip die Reaktionsprodukte schnell und effektiv vom Edukt abgetrennt werden. Mit Hilfe eines geeigneten, schwersiedenden Lösemittels wird verhindert, daß die Reaktion im Verdampferbereich abläuft, wobei das Lösemittel durch Verdampfung und Kondensation die Wärmeenergie vom Verdampfer in die Reaktionszone transportiert. Einer der beiden Produktströme wird als Seitenstrom entnommen, wobei durch den Rücklaufteiler ein energetisch optimiertes Profil eingestellt werden kann.

Überraschenderweise wurde gefunden, daß die Spaltung von Carbamidsäureestern mit hoher Raum-Zeit-Ausbeute direkt in einer Rektifizierkolonne derart betrieben werden kann, daß kein Carbamidsäureester mit den Heizflächen im Verdampfer in Berührung kommt und somit lange Betriebszeiten der Apparatur möglich sind. Im Gegensatz zu der in der DE-A 42 31 417 beschriebenen Kolonne und auch dem in EP-A 0 524 554 beschriebenem Reaktor ist nur eine geringe Ausschleusung von Sumpfinhalt notwendig, da an dieser Stelle weder Carbamidsäureester noch Spalt-bzw. Nebenprodukte analytisch nachweisbar sind. Die Verweilzeit der siedenden Flüssigkeit in der Reaktionszone ist im Gegensatz zur DE-A 42 31 417 nicht möglichst gering, sondern der Spaltkinetik angepaßt.

Gegenstand der Erfindung ist ein Verfahren zur thermischen Spaltung von Carbamidsäureestern, gegebenenfalls in Gegenwart eines Katalysators, zu Isocyanaten und Hydroxylverbindungen, dadurch gekennzeichnet, daß durch thermische Spaltung der den Isocyanaten entsprechenden Carbamidsäureester bei 150°C bis 400°C in einer Reaktivrektifikationskolonne mit parallel laufender Rektifikation die Spaltprodukte in eine aus mindestens 95 Gew.-% Alkohol bestehende Fraktion und eine aus mindestens 90 Gew.-% dem eingesetzten Carbamidsäureester entsprechende Isocyanat-Fraktion, wobei die Spaltung in Gegenwart eines Hochsieders durchgeführt wird, der für die Carbamidsäureester ein Lösemittel darstellt, gegenüber den Carbamidsäureestern und den Spaltprodukten weitgehend inert ist und die Wärmeenergie vom Verdampfer in die Reaktionszone im Abtriebsteil der Kolonne trägt, und daß der zu spaltende Carbamidsäureester gegebenenfalls in gelöster Form, mit einer Temperatur oberhalb des Schmelzpunktes des Esters oberhalb der Reaktionszone, aber unterhalb der Seitenstromentnahme in die Spaltkolonne geleitet wird, in deren Sumpf bei einem Betriebsdruck von 2 bis 1000 mbar und einer Temperatur von 150°C bis 400°C das Lösemittel am Sieden gehalten wird, wobei die Verweilzeit sowie der Stoff- und Wärmeübergang im Abtriebsteil und Mittelteil der Kolonne durch geeignete Einbauten so eingestellt werden, daß bereits hier die vollständige Spaltung des Carbamidsäureesters durchgeführt wird und gleichzeitig die Spaltprodukte durch kontinuierliche Rektifikation im Verstärkerteil am Kopf der Kolonne sowie die zweite Fraktion als Seitenstrom flüssig entnommen werden und über den Sumpfablauf kontinuierlich eine solche Menge des gegebenenfalls Verunreinigungen enthaltenden Hochsieders ausgeschleust wird, die in erster Nährung der Menge des Hochsieders entspricht, der in die Apparatur eingespeist wird.

Bei der Durchführung des erfindungsgemäßen Verfahrens kann die isocyanatreiche Fraktion einer Reindestillation unterzogen werden, wobei der Sumpf dieser Reindestillation dann ganz oder teilweise dem Zulauf der Spaltkolonne und/oder in einem Kreislaufverfahren in den Schritt der Urethansynthese zurückgeführt wird.

Bei dem erfindungsgemäßen Verfahren kann der Sumpfaustrag destillativ aufgearbeitet werden, wobei die leichter siedende Fraktion, die im wesentlichen das Lösemittel enthält, in den Zulauf der Spaltkolonne zugeführt wird.

Größere Ausschleusungsraten sind erst nötig, wenn der Reaktorzulauf bereits höhere Anteile von Nebenprodukten enthält und/oder wenn das Lösemittel in verstärktem Maße zur Bildung von Nebenprodukten neigt.

Durch das Verfahren gelingt es, eine vollständige, nebenproduktfreie Spaltung des Carbamidsäureesters noch im Abtriebsteil zu erreichen. Dies hat zur Folge, daß durch die vollständige Spaltung Ausbeuteverluste vermieden werden, daß die nachfolgende Aufarbeitung deutlich vereinfacht ist und daß Nebenreaktionen und Anbackungen im Sumpf vermieden werden, weil reagierende Produkte gar nicht in den Sumpf gelangen, so daß die Standzeiten entscheidend verlängert werden.

In einigen speziellen Fällen kann die Spaltung in Packungen selbst bei niedrigeren Hold-up und niedrigerer Verweilzeit wesentlich besser verlaufen als in Bodenkolonnen.

Wesentlich für die Erfindung ist der Ablauf der Reaktion als Reaktivrektifikation mit dem inerten Lösemittel. Der Lösemitteldampf steigt vom Verdampfer auf und stellt im Abtriebsteil durch Kondensation die Energie für die endotherme Reaktion sowie für die teilweise Verdampfung zur Verfügung. Das inerte Lösemittel sollte vorzugsweise einen engen Siedebereich und hohe thermische Stabilität aufweisen, wobei ein Reinstoff bevorzugt wird.

Bei den beim erfindungsgemäßen Verfahren einzusetzenden Carbamidsäureestern handelt es sich um Verbindungen der allgemeinen Formel R¹(NHCOOR²)ₙ.

In dieser Formel stehen
- R¹: für einen gegebenenfalls inerte Substituenten aufweisenden aliphatischen Kohlenwasserstoffrest mit insgesamt 4 bis 10 Kohlenstoffatomen, einen gegebenenfalls inerte Substituenten aufweisenden cycloaliphatischen Kohlenwasserstoffrest mit insgesamt 6 bis 15 Kohlenstoffatomen, einen gegebenenfalls inerte Substituenten aufweisenden araliphatischen Kohlenwasserstoffrest mit insgesamt 7 bis 10 Kohlenstoffatomen oder einen gegebenenfalls inerte Substituenten aufweisenden aromatischen Kohlenwasserstoffrest mit insgesamt 6 bis 15 Kohlenstoffatomen.
- R²: für einen aliphatischen Kohlenwasserstoffrest mit 1 bis 20 Kohlenstoffatomen, einen cycloaliphatischen Kohlenwasserstoffrest mit 5 bis 15 Kohlenstoffatomen oder einen aromatischen Kohlenwasserstoffrest mit 6 bis 15 Kohlenstoffatomen und
- n: für eine ganze Zahl von 2 bis 5.

Vorzugsweise werden beim erfindungsgemäßen Verfahren solche Carbamidsäureester der genannten Formel eingesetzt, für welche
- R¹: für einen aliphatischen Kohlenwasserstoffrest mit insgesamt 4 bis 12, insbesondere 5 bis 10 Kohlenstoffatomen, einen cycloaliphatischen Kohlenwasserstoffrest mit 6 bis 15 Kohlenstoffatomen, einem Xylylenrest oder einen aromatischen, gegebenenfalls Methylsubstituenten und/oder Methylenbrücken aufweisenden Kohlenwasserstoffrest mit insgesamt 6 bis 15 Kohlenstoffatomen steht,
- R²: für einen aliphatischen Kohlenwasserstoffrest mit 1 bis 6, insbesondere 1 bis 4 Kohlenstoffatomen, einen Cyclohexylrest oder einen Phenylrest steht, und
- n: für eine ganze Zahl von 2 bis 4 steht.

Beispiele für geeignete Carbamidsäureester sind
1-(Butoxycarbonylamino)-3,3,5-trimethyl-5-(butoxycarbonylamino-methyl)-cyclohexan,
1-(Methoxycarbonylamino)-3,3,5-trimethyl-5-(methoxycarbonylamino-methyl)-cyclohexan,
1-Methyl-2,4-bis-(methoxycarbonylamino)-benzol,
1-Methyl-2,6-bis-(methoxycarbonylamino)-benzol,
1-Methyl-2,4-bis-(butoxycarbonylamino)-benzol,
1-Methyl-2,6-bis-(butoxycarbonylamino)-benzol,
1,10-Bis-(methoxycarbonylamino)-decan,
1,12-Bis-(butoxycarbonylamino)-dodecan,
1,12-Bis-(methoxycarbonylamino)-dodecan,
1,12-Bis-(phenoxycarbonylamino)-dodecan,
1,3-Bis-(ethoxycarbonylamino-methyl)-benzol,
1,3-Bis-(methoxycarbonylamino)-benzol,
1,3-Bis-[(methoxycarbonylamino)-methyl]-benzol,
1,3,6-Tris-(methoxycarbonylamino)-hexan,
1,3,6-Tris-(phenoxycarbonylamino)-hexan,
1,4-Bis-(ethoxycarbonylamino)-butan,
1,4-Bis-(ethoxycarbonylamino)-cyclohexan,
1,5-Bis-(butoxycarbonylamino)-naphthalin,
1,6-Bis-(methoxycarbonylamino)-hexan,
1,6-Bis-(ethoxycarbonylamino)-hexan,
1,6-Bis-(butoxycarbonylamino)-hexan,
1,5-Bis-(methoxycarbonylamino)-pentan,
1,6-Bis-(methoxymethylcarbonylamino)-hexan,
1,8-Bis-(ethoxycarbonylamino)-octan,
1,8-Bis-(phenoxycarbonylamino)-4-(phenoxycarbonylaminmethyl)-octan,
2,2'-Bis-(4-propoxycarbonylamino-phenyl)-propan,
2,4'-Bis-(ethoxycarabonylamino)-diphenylmethan,
2,4-Bis-(methoxycarbonylamino)-cyclohexan,
4,4'-Bis-(ethoxycarbonylamino)-dicyclohexylmethan,
2,4'-Bis-(ethoxycarbonylamino)-diphenylmethan,
4,4'-Bis-(methoxycarbonylamino)-2,2'-dicyclohexylpropan,
4,4'-Bis-(methoxycarbonylamino)-biphenyl,
4,4'-Bis-(butoxycarbonylamino)-2,2'-dicyclohexylpropan,
4,4'-Bis-(phenoxycarbonylamino)-dicyclohexylmethan,
4,4'-Bis-Ophenoxycarbonylamino)-diphenylmethan,

Bei den genannten "Butoxy-Resten" handelt es sich um iso- und um n-Butoxy-Reste.

Als Lösemittel kommen u.a. Dibenzyltoluole, teilhydrierte Terphenyle und Phenoxybiphenyle bzw. deren Isomerengemische in Frage. Die Spaltprodukte steigen dampfförmig auf und werden somit unmittelbar der Reaktion in der Flüssigphase entzogen. Bei der Spaltung von mehrfunktionellen Carbamidsäureestern kann in dem Bereich zwischen Zulauf und Seitenabzug das teilgespaltene Zwischenprodukt niedergeschlagen und in den Reaktionsbereich zur vollständigen Spaltung rückgeführt werden. Im Verstärkerteil oberhalb des Seitenabzuges findet die Trennung zwischen der Hydroxylkomponente und dem Isocyanat statt. Hier ist aufgrund der Rückreaktion ein möglichst niedriger Flüssigkeits-Hold-up anzustreben.

Erfindungsgemäß kann zur Vermeidung von Rückreaktionen der Verstärker- und Mittelteil der Kolonne bei gegenüber dem Reaktionsteil in Abhängigkeit vom Sumpfdruck um bis zu 900 mbar vermindertem Druck betrieben werden, wobei die Trennung des Druckniveaus gegebenenfalls durch Abtrennung des Verstärker- und Mittelteils der Kolonne in einem gesonderten Apparat erfolgen kann.

Darüber hinaus kann der Rückreaktion auch durch einen nur im Verstärkerteil reduzierten Druck entgegengewirkt werden, der zu einer reduzierten Temperatur und damit zu verlangsamter Rückreaktion führt.

Als Apparatur sind Rektifizierkolonnen geeignet, die ein Abtriebsteil mit hinreichend hoher Verweilzeit und ein Verstärkerteil mit niedriger Verweilzeit aufweisen. Die Verweilzeit im Abtriebsteil ist auf die Spalt- und Stoffübergangskinetik abzustimmen und ist daher stark stoffsystemabhängig, sie beträgt 1 bis 1000 min, bevorzugt 5 bis 200 min und ist hier als Quotient von Flüssigkeits-Hold-up im Abtriebsteil und Zulauf-Volumenstrom der flüssigen Phase definiert. Druckverlustarme Packungen mit hohem Hold-up sind in Verbindung mit einem engsiedenden Lösemittel hierbei zu bevorzugen, da damit die Einstellung einer über den Reaktionsteil der Kolonne näherungsweise konstanten und über den Absolutdruck frei wählbaren Temperatur gelingt. Die Temperatur in der Reaktionszone ist bevorzugt in einem Temperaturbereich zu wählen, in dem einerseits die Reaktion ausreichend schnell verläuft und andererseits die Bildung von nicht recyclisierbaren Nebenprodukten noch nicht oder nur sehr schwach einsetzt.

Die Kolonne hat einen Rücklauf am Kolonnenkopf, mindestens einen Seitenabzug zur teilweisen oder vollständigen Entnahme der Flüssigphase sowie einen Sumpfablauf.

Als Verdampfer kommen alle gängigen Verdampfer zum Betreiben einer Kolonne in Betracht. Für einen dauerhaften Betrieb müssen die Heizflächen gut benetzt und umspült sein. Im Mittelteil und/oder im Abtriebsteil und/oder im Verstärkerteil der Kolonne sind als Einbauten Glockenböden, Siebböden oder geordnete bzw. ungeordnete Packungen möglich. Bevorzugt werden geordnete Packungen.

Der Reaktorzulauf besteht aus dem Carbamidsäureester, gegebenenfalls einem Katalysator und/oder einem inerten Lösemittel sowie gegebenenfalls aus den Nebenprodukten, die in einem Kreislaufverfahren entstehen, bei dem aus einem Amin, einer Carbonylquelle wie beispielsweise Oxide des Kohlenstoffs oder Kohlensäurederivate, vorzugsweise Harnstoff und/oder Carbamidsäureester bzw. Dialkylcarbonate und einer Hydroxylkomponente ein Carbamidsäureester hergestellt wird. Das recyclisierte Lösemittel kann gegebenenfalls teilweise oder vollständig direkt in den Sumpf der Kolonne eingebracht werden.

Der Zulaufstrom der Spaltkolonne setzt sich allgemein zusammen aus dem Hauptstrom aus dem vorhergeschalteten Verfahrensschritt sowie gegebenenfalls a) dem nebenproduktarmen Teilstrom, der aus dem Sumpfaustrag der Spaltkolonne nach Ausschleusung von höhersiedenden Nebenprodukten resultiert und gegebenenfalls b) aus dem Sumpfaustrag der Reinkolonne sowie gegebenenfalls c) Frisch-Lösungsmittel und gegebenenfalls d) Kopfprodukt der Reinkolonne. Der Zulauf wird auf eine Temperatur vorgewärmt, welche bis zu 250°C oberhalb der Schmelztemperatur des Carbamidsäureesters, vorzugsweise 50°C unterhalb der Reaktionstemperatur liegt. Der Zulauf wird oberhalb des Abtriebsteils in die Kolonne eingespeist.

Zur Erhöhung der Reaktionsgeschwindigkeit kann die Spaltung der Carbamidsäureester in Gegenwart von Katalysatoren erfolgen. Normalerweise sind bei dem erfindungsgemäßen Verfahren Katalysatoren nicht erforderlich. Falls Katalysatoren zugesetzt werden, so werden zweckmäßigerweise Mengen bis 10 Gew.-%, vorzugsweise bis 3 Gew.-%, bezogen auf das Gewicht des Carbamidsäureesters verwendet. Als Katalysatoren eignen sich z.B. Metalle, Metalloxide, anorganische oder organische Metallverbindungen sowie saure Zusätze. Beispielhaft können die in der US-A 3 919 279, US-A 4 388 246, DE-A 3 277 748, DE-A 3 248 018, DE-A 3 314 790, US-A 4 873 365, EP-A 323 514, EP-A 126 299, EP-A 566 925 und EP-A 568 782 genannten Stoffe Verwendung finden.

Es kann auch durch geeignet wirkende Packungs- oder Füllkörperoberflächen heterogen katalysiert werden.

Die Spaltkolonne wird mit einem Sumpfdruck von 2 bis 1000 mbar betrieben, bevorzugt wird der Druckbereich von 20 bis 200 mbar. Die Sumpftemperatur beträgt 150 bis 400°C, bevorzugt 220 bis 300°C. Sie richtet sich im wesentlichen nach der Siedetemperatur des Lösemittels und ist zudem so zu wählen, daß Nebenreaktionen des Carbamidsäureesters nur schwach auftreten. Das Rücklaufverhältnis am Kopf liegt zwischen 0,2 und 20, bevorzugt 2 bis 10. Das Rücklaufverhältnis an der Seitenstromentnahme liegt zwischen 0 und 40, bevorzugt zwischen 5 und 20.

Der Sumpfaustrag dient zur Ausschleusung von Nebenprodukten des gegebenenfalls nicht vollständig inerten Lösemittels sowie gegebenenfalls der Ausschleusung hochsiedender Verunreinigungen, die zusammen mit dem Carbamidsäureester zugefahren werden. Hierbei braucht nur die Menge an Lösemittel zu- bzw. abgeführt werden, wie zur Einhaltung einer vorgegebenen Nebenproduktkonzentration im Sumpf notwendig ist. Im Gegensatz zu den Verfahren der EP-A 0 524 554 und DE-A 42 31 417 erfolgt die vollständige Spaltung des Carbamidsäureesters bereits beim ersten Spaltkolonnendurchlauf, so daß kein Edukt im Kreis gefahren werden muß. In einem nachgeschalteten Apparat können hochsiedende Verunreinigungen aus dem Sumpfentnahmestrom auf an sich bekannte Weise, z.B. durch eine konventionelle Methode der Vakuumdestillation, vorzugsweise durch Dünnschichtdestillation und/oder durch Fallfilmdestillation ausgeschleust werden. Der lösemittelreiche Strom wird wieder auf die Spaltkolonne aufgegeben. Der isocyanatreiche Seitenstrom der Spaltkolonne wird anschließend einer Feindestillation unterworfen.

Der Sumpf der Feindestillation wird normalerweise dem Zulauf der Spaltkolonne beigemischt, bei einem größeren Anteil an höhermolekularen Reaktionsnebenprodukten kann der Sumpf auch ganz oder teilweise in die Stufe der Urethanherstellung zurückgeführt oder ausgeschleust werden.

### Beispiel

0,91 kg/h einer Mischung Hexamethylendi-n-butylurethan-1,6 (HDU-B) wurde zusammen mit 0,3 kg/h ortho-Phenoxybiphenyl (Gehalt nach GC >99 %) kontinuierlich in eine Spaltkolonne eingebracht. Die Aufgabe von HDU-B und von ortho-Phenoxybiphenyl erfolgte oberhalb des Abtriebsteils.

Die Kolonne besteht aus einem Verdampfer mit 4 waagerecht angeordneten Heizkerzen. Darüber befinden sich im Abtriebsteil auf einer Länge von 8,1 m eine geordnete Packung mit einem Durchmesser von 70 mm und einem Flüssigkeits-Hold-up von insgesamt rund 1500 ml. Oberhalb dieser Reaktionszone befindet sich der Zulauf, wobei das HDU-B mit 120°C und das Lösemittel mit 160°C zudosiert wird. Der Mittelteil der Kolonne hat 70 mm Durchmesser und ist über eine effektive Höhe von 990 mm mit Gewebe-Packungen bestückt. Darüber folgt die Seitenstrom-Entnahme und 850 mm effektive Gewebe-Packung mit 50 mm Durchmesser. Der Kopf der Kolonne besteht aus dem Flüssigkeitsteiler und einem wassergekühlten Kondensator. Die Kolonne ist isoliert. Das Kopfrücklaufverhältnis betrug im Beispiel 7, das Seitenstromrücklaufverhältnis 10. Der Kopfdruck betrug in diesem Beispiel 85 mbar, die Sumpftemperatur 260°C. Dem Kolonnensumpf wurden 0,3 kg/h Flüssigkeit entnommen, die gemäß Supercritcal Fluid Chromatography (SFC) nur den Wärmeträger enthielten, HDU-B oder dessen Folgeprodukte waren nicht nachweisbar (Nachweisgrenze 0,1 %). Den gleichen Befund erbrachte die IR-Analytik. Ebenso rein war die Flüssigkeit, die von der Destillationskolonne in den Sumpf abtropfte und von der Probe entnommen wurde, auch hier wurden weder über SFC- noch über IR-Analytik Verunreinigungen gefunden. Der Seitenstrom von 0,48 kg/h bestand aus 98,2 Gew.-% HDI, 1,6 Gew.-% halbgespaltenes Produkt der Formel (I)

BuOCONH-(CH₂)₆-NCO (I).

0,1 Gew.-% ortho-Phenoxybiphenyl und 0,1 Gew.-% BuOH. Der Kopf-Entnahmestrom betrug 0,43 kg/h und hatte eine Zusammensetzung von 99,5 % BuOH, 0,2 Gew.-% HDU-B und 0,3 Gew.-% (I). Die Ausbeute (maximal mögliche Menge HDI abzüglich Verlusten in Sumpf und Kopf) bei diesem Versuch betrug deutlich über 99 %. Hierbei ist der Gehalt an (I) im Seitenstrom nicht als Verlust gewertet, da (I) über den Rücklauf von der Reinkolonne zurückgewonnen werden kann. Die Ausbeute kann weiter erhöht werden, wenn das Kopfprodukt recyclisiert wird.

## Patentansprüche

1. Verfahren zur thermischen Spaltung von Carbamidsäureestern, gegebenenfalls in Gegenwart eines Katalysators, zu Isocyanaten und Hydroxylverbindungen, **dadurch gekennzeichnet, daß** durch thermische Spaltung der den Isocyanaten entsprechenden Carbamidsäureester bei 150°C bis 400°C in einer Reaktivrektifikationskolonne mit gleichzeitig laufender Rektifikation die Spaltprodukte in eine aus mindestens 95 Gew.-% Alkohol bestehende Fraktion und eine aus mindestens 90 Gew.-% dem eingesetzten Carbamidsäureester entsprechende Isocyanat-Fraktion, wobei die Spaltung in Gegenwart eines Hochsieders durchgeführt wird, der für die Carbamidsäureester ein Lösemittel darstellt, gegenüber den Carbamidsäureestern und den Spaltprodukten weitgehend inert ist und die Wärmeenergie vom Verdampfer in die Reaktionszone im Abtriebsteil der Kolonne trägt und der zu spaltende Carbamidsäureester gegebenenfalls in gelöster Form, mit einer Temperatur oberhalb des Schmelzpunktes des Esters oberhalb der Reaktionszone, aber unterhalb der Seitenstromentnahme in die Spaltkolonne geleitet wird, in deren Sumpf bei einem Betriebsdruck von 2 bis 1000 mbar und einer Temperatur von 150°C bis 400°C das Lösemittel am Sieden gehalten wird und die Verweilzeit sowie der Stoff- und Wärmeübergang im Abtriebsteil und Mittelteil der Kolonne durch geeignete Einbauten so eingestellt werden, daß bereits hier die vollständige Spaltung des Carbamidsäureesters durchgeführt wird und gleichzeitig die Spaltprodukte durch kontinuierliche Rektifikation im Verstärkerteil am Kopf der Kolonne sowie die zweite Fraktion als Seitenstrom flüssig entnommen werden und über den Sumpfablauf kontinuierlich eine solche Menge des gegebenenfalls Verunreinigungen enthaltenden Hochsieders ausgeschleust wird, die in erster Nährung der Menge des Hochsieders entspricht, der in die Apparatur eingespeist wird.

2. Verfahren gemäß Anspruch 1, **dadurch gekennzeichnet, daß** die isocyanatreiche Fraktion einer Reindestillation unterzogen wird, wobei der Sumpf dieser Reindestillation ganz oder teilweise dem Zulauf der Spaltkolonne und/oder in einem Kreislaufverfahren dem Schritt der Urethansynthese zurückgeführt wird.

3. Verfahren gemäß Anspruch 1, **dadurch gekennzeichnet, daß** der Sumpfaustrag destillativ aufgearbeitet wird, und die leichtersiedende Fraktion, die im wesentlichen das Lösemittel enthält ganz oder teilweise in den Zulauf der Spaltkolonne zurückgeführt wird.

4. Verfahren gemäß Anspruch 1, **dadurch gekennzeichnet, daß** man als Hochsieder die isomeren Dibenzyltoluole, teilhydrierte Terphenyle und Phenoxybiphenyle bzw. deren Isomerengemische verwendet.

5. Verfahren gemäß Anspruch 1, **dadurch gekennzeichnet, daß** Verstärker und gegebenenfalls Mittelteil der Kolonne zur Vermeidung von Rückreaktionen bei gegenüber dem Reaktionsteil vermindertem Druck betrieben werden, wobei die Trennung des Druckniveaus auch durch Abtrennung des Verstärker- und gegebenenfalls Mittelteils der Kolonne in einem gesonderten Apparat erfolgen kann.

6. Verfahren gemäß Anspruch 1, wobei die Reaktion in Gegenwart eines Katalysators durchgeführt wird.

## Claims

1. A process for the thermal cracking of carbamidic acid esters, optionally in the presence of a catalyst, to form isocyanates and hydroxyl compounds, **characterised in that** by thermal cracking of carbamidic acid esters corresponding to the isocyanates at 150°C to 400°C in a reactive rectification column with rectification proceeding simultaneously the cracking products are separated into a fraction consisting of at least 95 % by weight alcohol and into an isocyanate fraction corresponding to at least 90 % by weight of the carbamidic acid ester used, wherein cracking is conducted in the presence of a high-boiling substance which constitutes a solvent for the carbamidic acid esters, which is substantially inert to the carbamidic acid esters and the cracking products, and which carries the heat energy from the evaporator into the reaction zone in the stripping part of the column, and the carbamidic acid ester to be cracked is fed into the cracking column, optionally in dissolved form, at a temperature above the melting point of the ester, above the reaction zone but below the side stream take-off, in the bottom of which cracking column the solvent is kept boiling at an operating pressure of 2 to 1000 mbar and at a temperature of 150°C to 400°C, and the dwell time and the mass and heat transfer in the stripping part and middle part of the column are adjusted by suitable baffles so that complete cracking of the carbamidic acid ester is already effected here and the cracking products are taken off simultaneously by continuous rectification in the enrichment part at the top of the column and the second fraction is taken off in liquid form as a side stream, and the high-boiling substance, which possibly contains impurities, is transferred out via the bottom discharge in an amount which corresponds to a first approximation to the amount of high-boiling substance which is fed into the apparatus.

2. A process according to claim 1, **characterised in that** the isocyanate-rich fraction is subjected to a purification distillation, wherein the bottoms of this purification distillation are then recycled completely or partially to the feed to the cracking column and/or into the urethane synthesis step in a cyclic process.

3. A process according to claim 1, **characterised in that** bottom discharge is worked up by distillation, and the lower-boiling fraction, which essentially contains the solvent, is recycled completely or partially to the feed of the cracking column.

4. A process according to claim 1, **characterised in that** isomeric dibenzyl toluenes, partially hydrogenated terphenyls and phenoxybiphenyls, or mixtures of isomers thereof, are used as high-boiling substances.

5. A process according to claim 1, **characterised in that** in order to prevent back-reactions the enrichment part and optionally the middle part of the column are operated at a pressure which is reduced compared with the reaction part, wherein the separation of the pressure level may also be effected by separating the enrichment part and optionally the middle part of the column in a separate apparatus.

6. A process according to claim 1, wherein the reaction is conducted in the presence of a catalyst.

## Revendications

1. Procédé de clivage thermique d'esters carbamiques, facultativement en présence d'un catalyseur, en isocyanates et composés hydroxylés, **caractérisé en ce que** par le clivage thermique de l'ester carbamique conduisant à l'isocyanate de 150°C à 400°C, dans une colonne de rectification réactive avec une rectification se déroulant en même temps les produits de clivage sont scindés en une fraction constituée d'au moins 95% en poids d'alcool et une fraction isocyanate correspondant à au moins 90% de l'ester carbamique mis en oeuvre, où le clivage est réalisé en présence d'un composé à point d'ébullition élevé qui représente un solvant pour l'ester carbamique, qui est essentiellement inerte par rapport à l'ester carbamique et aux produits de clivage et qui transporte l'énergie thermique de l'évaporateur dans la zone de réaction, dans la partie de rectification-strippage de la colonne et **en ce que** l'ester carbamique à cliver est conduit dans la colonne de clivage, facultativement sous forme dissoute, à une température supérieure au point de fusion de l'ester, au-dessus de la zone de réaction mais en dessous du prélèvement du courant latéral, colonne dans le bas de laquelle le solvant est maintenu à ébullition à une pression de fonctionnement allant de 2 à 1000 bar et à une température allant de 150°C à 400°C, le temps de séjour ainsi que le transfert de matière et de chaleur dans la partie de rectification-strippage et la partie moyenne de la colonne étant ajustés par une structure interne appropriée telle que le clivage complet de l'ester carbamique y soit déjà effectué et simultanément, les produits de clivage sont prélevés sous forme liquide, par rectification continue dans la partie de rectification-renforcement à la tête de la colonne, ainsi que la deuxième fraction en tant que courant latéral et via la sortie du bas, on écluse en continu une quantité du composé à point d'ébullition élevé contenant facultativement des impuretés, telle que dans la première alimentation, la quantité du composé à point d'ébullition élevé correspond à celle qui est injectée dans l'appareil.

2. Procédé suivant la revendication 1, **caractérisé en ce que** la fraction riche en isocyanate est soumise à une distillation de purification, où le bas de colonne de cette distillation de purification est recyclé complètement ou partiellement dans l'entrée de la colonne de clivage et/ou dans un procédé en cycle fermé, dans l'étape de synthèse de l'uréthanne.

3. Procédé suivant la revendication 1, **caractérisé en ce que** l'évacuation du bas de la colonne est traitée par distillation, et la fraction plus volatile, qui contient essentiellement le solvant, est complètement ou partiellement recyclée dans l'entrée de la colonne de clivage.

4. Procédé suivant la revendication 1, **caractérisé en ce que** l'on utilise comme composé à ébullition élevée, les isomères du dibenzyltoluène, des terphényles partiellement hydrogénés et le phénoxybiphényle ou les mélanges de leurs isomères.

5. Procédé suivant la revendication 1, **caractérisé en ce que** la partie de rectification-renforcement et facultativement, la partie moyenne de la colonne sont mis en oeuvre à pression réduite par rapport à la partie de réaction, pour éviter les réactions en retour, où la séparation des niveaux de pression peut se faire par séparation des parties de rectification-renforcement et facultativement, moyenne de la colonne dans un appareil séparé.

6. Procédé suivant la revendication 1, où la réaction est réalisée en présence d'un catalyseur.
